(19) Europäisches Patentamt
European Patent Office
Office européen des brevets

(11) Veröffentlichungsnummer: **0 195 939 B1**

(12) # EUROPÄISCHE PATENTSCHRIFT

(45) Veröffentlichungstag der Patentschrift: **12.06.91**

(21) Anmeldenummer: **86102386.9**

(22) Anmeldetag: **24.02.86**

Die Akte enthält technische Angaben, die nach dem Eingang der Anmeldung eingereicht wurden und die nicht in dieser Patentschrift enthalten sind.

(51) Int. Cl.5: **C07D 487/14**, C07D 495/22, A61K 31/55, //(C07D487/14, 243:00,235:00,205:00), (C07D487/14,243:00,235:00, 209:00),(C07D495/22,333:00, 243:00,235:00,209:00), (C07D495/22,333:00,243:00, 235:00,205:00)

(54) **Imidazodiazepin-Derivate.**

(30) Priorität: **28.02.85 CH 902/85**

(43) Veröffentlichungstag der Anmeldung:
**01.10.86 Patentblatt 86/40**

(45) Bekanntmachung des Hinweises auf die Patenterteilung:
**12.06.91 Patentblatt 91/24**

(84) Benannte Vertragsstaaten:
**AT BE CH DE FR GB IT LI LU NL SE**

(56) Entgegenhaltungen:
**EP-A- 0 100 906**
**EP-A- 0 150 040**

(73) Patentinhaber: **F. HOFFMANN-LA ROCHE AG**
**Postfach 3255**
**CH-4002 Basel(CH)**

(72) Erfinder: **Hunkeler, Walter, Dr.**
**Im Stigler 32**
**CH-4312 Magden(CH)**
Erfinder: **Kyburz, Emilio, Dr.**
**Unterer Rebbergweg 127**
**CH-4153 Reinach(CH)**

(74) Vertreter: **Lederer, Franz, Dr. et al**
**Lederer, Keller & Riederer Patentanwälte**
**Lucile-Grahn-Strasse 22**
**W-8000 München 80(DE)**

EP 0 195 939 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JOURNAL OF HETEROCYCLIC CHEMISTRY, Band 15, Nr. 5, August 1978, Seiten 855-858, Hetero Corp.; A. WALSER et al.: "Quinazolines and 1,4-benzodiazepines. LXXXVII (1). Synthesis of 1- and 3-phenylimidazo [1,5-alpha][1,4]benzodiazepines"

GOODMAN and GILMAN'S "The pharmacological basis of therapeutics", 7. Ausgabe, Seiten 339-351, Macmillan Publishing Co., New York, USA; S.C. HARVEY: "Hypnotics and sedatives"

MEDICINAL CHEMISTRY, 3. Ausgabe, Teil I, A. Burger, Wiley-Interscience

## Beschreibung

Die vorliegende Erfindung betrifft Imidazodiazepin-Derivate. Im speziellen betrifft sie Imidazodiazepin-Derivate der allgemeinen Formel

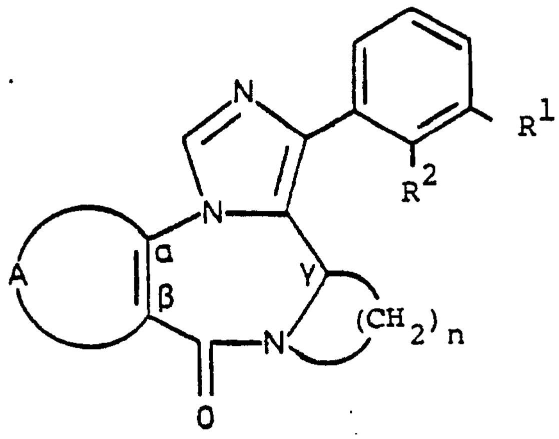

I

worin A zusammen mit den mit α und β bezeichneten Kohlenstoffatomen die Gruppe

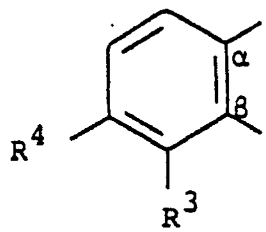

(a)

oder

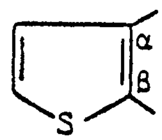

(b)

eines von $R^1$ und $R^2$ Wasserstoff und das andere Wasserstoff, Halogen oder Trifluormethyl, $R^3$ und $R^4$ je Wasserstoff oder Halogen und n die Zahl 2 oder 3 bedeuten, wobei die Verbindungen bezüglich des mit γ bezeichneten Kohlenstoffatoms die (S)- oder (R,S)-Konfiguration aufweisen,
und pharmazeutisch annehmbare Säureadditionssalze davon.

Diese Verbindungen sind neu und zeichnen sich durch wertvolle pharmakodynamische Eigenschaften aus.

Gegenstand der vorliegenden Erfindung sind Verbindungen der obigen Formel I und pharmazeutisch annehmbare Säureadditionssalze davon als solche und als pharmazeutische Wirkstoffe, die Herstellung dieser Verbindungen und Salze, Arzneimittel, enthaltend eine Verbindung der Formel I oder ein pharmazeutisch annehmbares Säureadditionssalz davon, die Herstellung solcher Arzneimittel und die Verwendung von Verbindungen der Formel I und ihrer pharmazeutisch annehmbaren Säureadditionssalze bei der Bekämpfung oder Verhütung von Krankheiten (insbesondere von Konvulsionen, Angstzuständen, Muskelspannungen, Spannungszuständen und Schlaflosigkeit) bzw. zur Herstellung von Arzneimitteln, insbesondere von anitkonvulsiv, anxiolytisch, muskelrelaxierend und sedativ-hypnotisch wirksamen Arzneimitteln.

In der Europäischen Patentpublikation Nr. 150 040 werden unter anderem den Verbindungen der Formel I entsprechende Imidazobenzo- und Imidazothienodiazepinderivate offenbart, welche am ankondensierten Imidazolring an Stelle der gegebenenfalls substituierten Phenylgruppe eine gegebenenfalls substituierte aromatische heterocyclische Gruppe, beispielsweise eine Thienylgruppe, tragen. Jene Verbindungen besitzen ebenfalls antikonvulsive, anxiolytische, muskelrelaxierende und sedativ-hypnotische Eigenschaften.

Der Ausdruck "nieder" bezeichnet Reste und Verbindungen mit höchstens 7, vorzugsweise höchstens 4 Kohlenstoffatomen. Der Ausdruck "Alkyl" bezeichnet geradkettige oder verzweigte gesättigte Kohlenwasserstoffreste, wie Methyl, Aethyl, n-Propyl, Isopropyl, n-Butyl, s-Butyl, t-Butyl und dgl. Der Ausdruck "Aryl" bezeichnet monocyclische aromatische Kohlenwasserstoffreste, welche durch niederes Alkyl, niederes

Alkoxy, Halogen usw. substituiert sein können. Der Ausdruck "Aralkyl" bezeichnet einen durch Aryl substituierten Alkylrest im Sinne der vorstehenden Definitionen. Der Ausdruck "Alkoxy" bezeichnet über ein Sauerstoffatom gebundene Alkylgruppen, wie Methoxy, Aethoxy und dgl. Die Ausdrücke "Alkylthio" und "Aralkylthio" bezeichnen über ein Schwefelatom gebundene Alkyl- bzw. Aralkylgruppen.

Wenn das Symbol A in Formel I die Grupppe (a) bedeutet, dann bedeuten vorzugsweise $R^3$ Halogen (insbesondere Chlor oder Brom) und $R^4$ Wasserstoff. $R^1$ bedeutet vorzugsweise Halogen (insbesondere Fluor, Chlor oder Brom) oder Trifluormethyl, und $R^2$ bedeutet vorzugsweise Wasserstoff. Das in Formel I mit $\gamma$ bezeichnete Kohlenstoffatom weist vorzugsweise die (S)-Konfiguration auf.

Im Rahmen der vorliegenden Erfindung bevorzugte Vertreter der durch die allgemeinen Formel I definierten Stoffklasse sind:

(S)-8-Chlor-1-(m-chlorphenyl)-11,12,13,13a-tetrahydro-9H-imidazo[1,5-a]pyrrolo[2,1-c][1,4]-benzodiazepin-9-on,

(S)-8-Chlor-1-(m-fluorphenyl)-11,12,13,13a-tetrahydro-9H-imidazo[1,5-a]pyrrolo[2,1-c][1,4]benzodiazepin-9-on und

(S)-8-Brom-1-(m-chlorphenyl)-11,12,13,13a-tetrahydro-9H-imidazo[1,5-a]pyrrolo[2,1-c][1,4]-benzodiazepin-9-on.

Weitere repräsentative Verbindungen der Formel I sind:

(S)-8-Chlor-11,12,13,13a-tetrahydro-1-phenyl-9H-imidazo-[1,5-a]pyrrolo[2,1-c][1,4]benzodiazepin-9-on,

(S)-1-(m-Chlorphenyl)-11,12,13,13a-tetrahydro-9H-imidazo-[1,5-a]pyrrolo[2,1-c][1,4]benzodiazepin-9-on,

(S)-8-Chlor-1-(m-chlorphenyl)-7-fluor-11,12,13,13a-tetrahydro-9H-imidazo-[1,5-a]pyrrolo[2,1-c][1,4]-benzodiazepin-9-on,

(S)-8-Chlor-11,12,13,13a-tetrahydro-1-($\alpha,\alpha,\alpha$-trifluor-m-tolyl)-9H-imidazo-[1,5-a]pyrrolo[2,1-c][1,4]-benzodiazepin-9-on,

(S)-1-(m-Chlorphenyl)-7-fluor-11,12,13,13a-tetrahydro-9H-imidazo-[1,5-a]pyrrolo[2,1-c][1,4]-benzodiazepin-9-on,

(S)-1-(m-Bromphenyl)-8-chlor-11,12,13,13a-tetrahydro-9H-imidazo-[1,5-a]pyrrolo[2,1-c][1,4]-benzodiazepin-9-on,

(S)-1-(m-Bromphenyl)-10,11,12,12a-tetrahydro-8H-imidazo-[5,1-c]pyrrolo[1,2-a]thieno[3,2-e][1,4]-diazepin-8-on,

(S)-8-Chlor-1-(m-chlorphenyl)-12,12a-dihydro-9H-azeto-[2,1-c]imidazo[1,5-a][1,4]benzodiazepin-9-on und

(S)-8-Chlor-1-(o-chlorphenyl)-11,12,13,13a-tetrahydro-9H-imidazo-[1,5-a]pyrrolo[2,1-c][1,4]-benzodiazepin-9-on.

Die Verbindungen der Formel I und ihre pharmazeutisch annehmbaren Säureadditionssalze können erfindungsgemäss dadurch hergestellt werden, dass man eine Verbindung der allgemeinen Formel

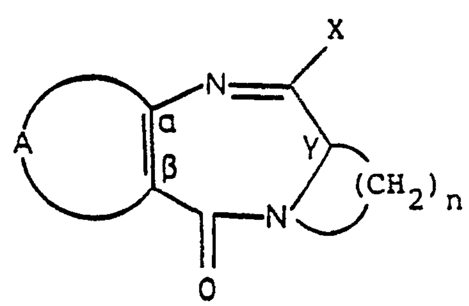

II

worin A und n obige Bedeutung besitzen und X eine Abgangsgruppe bedeutet, in Gegenwart einer Base mit einem Isonitril der allgemeinen Formel

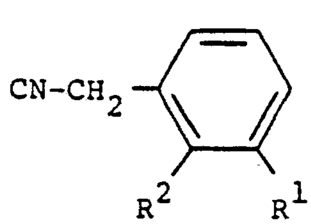

III

worin $R^1$ und $R^2$ obige Bedeutung besitzen,
umsetzt und erwünschtenfalls eine erhaltene Verbindung der allgemeinen Formel I in ein pharmazeutisch annehmbares Säureadditionssalz überführt.

Erfindungsgemäss können Verbindungen der allgemeinen Formel I demnach aus Verbindungen der allgemeinen Formel II und Isocyanessigestern der allgemeinen Formel III hergestellt werden. Die in Formel II durch das Symbol X bezeichnete Abgangsgruppe ist beispielsweise eine leicht abspaltbare Phosphinylgruppe, z.B. eine Gruppe der Formel

$-OP(O)(OR^5)_2$ oder $-OP(O)(NR^6R^7)_2$

worin $R^5$ niederes Alkyl oder Aryl (wie Phenyl) und
$R^6$ und $R^7$ je niederes Alkyl, Allyl, Aryl (wie Phenyl) oder zusammen mit dem Stickstoffatom einen unsubstituierten oder substituierten heterocyclischen Ring mit 3-8 Gliedern (wie Morpholin) bedeuten,
ein Halogenatom, eine niedere Alkylthiogruppe, eine niedere Aralkylthiogruppe, eine niedere N-Nitrosoalkylaminogruppe, eine niedere Alkoxygruppe, eine Mercaptogruppe und dergleichen (wenn X eine Mercaptogruppe bedeutet, so handelt es sich bei der entsprechenden Verbindung der Formel II um die Iminothiol-Form des entsprechenden Thiolactams). Die Umsetzung der Verbindungen der Formeln II und III erfolgt in einem inerten Lösungsmittel, wie Dimethylformamid, Hexamethylphosphorsäuretriamid, Dimethylsulfoxid, Tetrahydrofuran oder irgend einem anderen geeigneten organischen Lösungsmittel und in Gegenwart einer Base, die genügend stark basisch ist, um das Anion des Isocyanessigesters der Formel III zu bilden. Als Basen eignen sich Alkalimetallalkoxide, wie Natriummethoxid oder Kalium-t-butoxid, Alkalimetallhydride, wie Natriumhydrid, Alkalimetallamide, wie Lithiumamid oder Lithiumdiisopropylamid, tertiäre Amine, wie Triäthylamin, und dergleichen. Die Reaktionstemperaratur liegt zweckmässigerweise zwischen etwa -40 °C und etwa Raumtemperatur.

Erwünschtenfalls können Verbindungen der allgemeinen Formel I erfindungsgemäss in pharmazeutisch annehmbare Säureadditionssalze übergeführt werden. Die Herstellung von solchen pharmazeutisch annehmbaren Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate und dergleichen.

Die als Ausgangsstoffe verwendeten Verbindungen der Formeln II und III sind bekannt oder können in Analogie zu den bekannten Vertretern dieser Stoffklassen hergestellt werden. Die Verbindungen der Formel II werden in der Regel nicht gefasst, sondern in situ mit den Verbindungen der Formel III umgesetzt.

Wie eingangs erwähnt sind die Verbindungen der Formel I neu; sie besitzen äusserst wertvolle pharmakodynamische Eigenschaften und weisen nur eine geringe Toxizität auf. Sie besitzen als gemeinsames Merkmal eine ausgeprägte Affinität zu den zentralen Benzodiazepin-Rezeptoren und haben ausgeprägte anxiolytische, antikonvulsive, muskelrelaxierende und sedativ-hypnotische Eigenschaften.

Die Affinität von Verbindungen der allgemeinen Formel I zu den zentralen Benzodiazepin-Rezeptoren wurde nach der in Life Science 20, 2101-2110 (1977) und Science 198, 849-851 (1977) beschriebenen Methode festgestellt. Nach dieser Methode ermittelt man die Hemmung der Bindung von tritiiertem Diazepam an die spezifischen Benzodiazepin-Rezeptoren im cerebralen Cortex durch die jeweiligen Testsubstanzen. Man bezeichnet als $IC_{50}$ ("50% inhibiting concentration") diejenige Konzentration der jeweiligen Testsubstanz, welch eine 50-proz. Hemmung der spezifischen Bindung des tritiierten Diazepams an die spezifischen Benzodiazepin-Rezeptoren im cerebralen Cortex bewirkt.

Die zentralen Eigenschaften der erfindungsgemässen Verbindungen der Formel I können beispielsweise im nachfolgend beschriebenen und für die Erfassung antikonvulsiver Eigenschaften allgemein anerkannten Antipentetrazoltest nachgewiesen werden.

In diesem Tierversuch verabreicht man 60-80 g schweren, weiblichen Ratten die zu testende Verbindung oral und 30 Minuten später 120 mg/kg i.p. Pentetrazol, das in ungeschützten Versuchstieren 1 bis 4 Minuten nach der Injektion Emprosthotonus und tonische Streckungen der vorderen und/oder hinteren Gliedmassen bewirkt. Man verwendet zehn Versuchstiere pro Dosis Testsubstanz. Nach Auszählen der geschützten Versuchstiere ermittelt man die $ED_{50}$ nach der Probit-Methode. Die $ED_{50}$ ist diejenige Dosis, welche 50% der Versuchstiere von den durch Pentetrazol bewirkten krampfartigen Anfällen schützt.

In der nachfolgenden Tabelle werden die Resultate dargestellt, welche mit repräsentativen Vertretern der durch die allgemeinen Formel I definierten Verbindungsklasse in den vorstehend geschilderten Versuchen erhalten worden sind. Ausserdem enthält die Tabelle Angaben über die akute Toxizität einiger dieser Verbindungen ($DL_{50}$ in mg/kg bei einmaliger oraler Verabreichung an Mäuse).

| Verbindung | $IC_{50}$ in nM/l | Antipentetrazol-Test, $ED_{50}$ in mg/kg p.o. | Toxizität $DL_{50}$ in mg/kg p.o. |
|---|---|---|---|
| A | 4,2 | 2,1 | 2500-5000 |
| B | 40 | 4,0 | 2500-5000 |
| C | 2,0 | 1,0 | >5000 |

A: (S)-8-Chlor-1-(m-chlorphenyl)-11,12,13,13a-tetrahydro-9H-imidazo[1,5-a]pyrrolo[2,1-c]-benzodiazepin-9-on

B: (S)-8-Chlor-11,12,13,13a-tetrahydro-1-(α,α,α-trifluor-m-tolyl)-9H-imidazo[1,5-a]pyrrolo[2,1-c][1,4]-benzodiazepin-9-on

C: (S)-8-Brom-1-(m-chlorphenyl)-11,12,13,13a-tetrahydro-9H-imidazo[1,5-a]pyrrolo[2,1-c][1,4]-benzodiazepin-9-on

Die Verbindungen der Formel I und ihre pharmazeutisch annehmbaren Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze, zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend eine Verbindung der Formel I oder ein pharmazeutisch annehmbares Säureadditionssalz davon und ein therapeutisch inertes Excipiens, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der Formel I oder pharmazeutisch annehmbare Säureadditionssalze davon und gegebenenfalls einen oder mehrere andere therapeutisch wertvolle Stoffe zusammen mit einem oder mehreren therapeutisch inerten Excipientien in eine galenische Darreichungsform bringt.

Wie eingangs erwähnt, können die Verbindungen der Formel I und ihre pharmazeutisch annehmbaren Säureadditionssalze erfindungsgemäss bei der Bekämpfung bzw. Verhütung von Krankheiten verwendet werden, und zwar insbesondere bei der Bekämpfung von Konvulsionen, Angstzuständen Muskelspannungen, Spannungszuständen und Schlaflosigkeit. Die Dosierung kann innerhalb weiter Grenzen variieren und ist natürlich in jedem einzelnen Fall den individuellen Gegebenheiten anzupassen. Im allgemeinen kommt bei oraler Verabreichung eine Tagesdosis von etwa 1 mg bis 100 mg in Frage.

Ebenfalls Gegenstand der Erfindung ist, wie eingangs erwähnt, die Verwendung der Verbindungen der Formel I und ihrer pharmazeutisch annehmbaren Säureadditionssalze zur Herstellung von Arzneimitteln, insbesondere von Arzneimitteln mit anxiolytischen, antikonvulsiven, muskelrelaxierenden und sedativ-hypnotischen Eigenschaften.

Die nachfolgenden Beispiele sollen die vorliegende Erfindung näher erläutern, ihren Umfang jedoch in keiner Weise beschränken. Sämtliche Temperaturen sind in Celsiusgraden angegeben.

Beispiel 1

Man versetzt eine Suspension von 9,57 g (38,2 mMol) (S)-6-Chlor-1,2,3,11a-tetrahydro-5H -pyrrolo[2,1-c][1,4]-benzodiazepin-5,11(10H)-dion in 25 ml trockenem N,N-Dimethylformamid bei -30° bis -20° mit 1,60 g (36,6 mMol) Natriumhydrid (55-proz. Oeldispersion), rührt noch während 25 minuten bei obiger Temperatur und tropft dann bei -65° eine Lösung von 9,75 g (36,3 mMol) Phosphorsäure-diphenylesterchlorid in 8 ml trockenem N,N-Dimethylformamid zu. Man rührt 15 Minuten im Aceton/Trockeneisbad und gibt bei -70° eine Lösung von 5,79 g (38,2 mMol) 3-Chlorbenzylisocyanid in 4 ml trockenem N,N-Dimethylformamid und anschliessend bei 70° bis -45° eine Lösung von 4,28 g (38,2 mMol) Kalium-t-butylat in 10 ml trockenem N,N-Dimethylformamid zu. Man lässt auf Raumtemperatur erwärmen und neutralisiert mit 2 ml Eisessig. Man giesst auf 250 ml Wasser und extrahiert fünfmal mit Methylenchlorid. Die vereinigten organischen Auszüge werden dreimal mit Wasser gewaschen, über Magnesiumsulfat getrocknet und eingedampft. Das Rohprodukt wird unter Eluieren mit Essigester an Kieselgel chromatographiert und aus Methylenchlorid und Essigester umkristallisiert. Man erhält (S)-8-Chlor-1-(m-chlorphenyl)-11,12,13,13a-tetrahydro-9H-imidazo[1,5-a]pyrrolo[2,1-c][1,4]benzodiazepin-9-on vom Schmelzpunkt 244-245°.

Beispiel 2

Man versetzt eine Suspension von 9,57 g (38,2 mMol) (S)-6-Chlor-1,2,3,11a-tetrahydro-5H -pyrrolo[2,1-c][1,4]-benzodiazepin-5,11(10H)-dion in 35 ml trockenem N,N-Dimethylformamid bei -30° bis -20° mit 1,60 g (36,6 mMol) Natriumhydrid (55-proz. Oeldispersion), rührt noch während 45 Minuten bei obiger Temperatur und tropft dann bei -70° eine Lösung von 9,75 g (36,3 mMol) Phosphorsäure-diphenylesterchlorid in 8 ml trockenem N,N-Dimethylformamid zu. Man rührt noch während 45 Minuten im Aceton/Trockeneisbad und tropft bei -70° bis -60° eine Lösung von 4,44 g (37,9 mMol) Benzylisocyanid in 5 ml trockenem N,N-Dimethylformamid und anschliessend eine Lösung von 4,28 g (38,2 mMol) Kalium-t-butylat in 12 ml trockenem N,N-Dimethylformamid zu. Man lässt auf Raumtemperatur erwärmen und neutralisiert durch Zugabe von 2 ml Eisessig. Man giesst auf 250 ml Wasser und extrahiert fünfmal mit Methylenchlorid. Man wäscht die vereinigten organischen Auszüge dreimal mit Wasser, trocknet sie über Magnesiumsulfat und dampft sie ein. Durch Chromatographieren des Rückstands an Kieselgel unter Eluieren mit Essigester und anschliessende Umkristallisation aus Essigester erhält man (S)-8-Chlor-11,12,13,13a-tetrahydro-1-phenyl -9H-imidazo[1,5-a]pyrrolo[2,1-c][1,4]benzodiazepin-9-on vom Schmelzpunkt 233-234°.

Beispiel 3

Man versetzt eine Suspension von 12,53 g (50 mMol) (S)-6-Chlor-1,2,3,11a-tetrahydro -5H-pyrrolo[2,1-c][1,4]-benzodiazepin-5,11(10H)-dion in 40 ml trockenem N,N-Dimethylformamid bei -30° bis -20° mit 2,18 g (50 mMol) Natriumhydrid (55-proz. Oeldispersion), rührt noch während 45 Minuten bei obiger Temperatur und tropft dann bei -65° eine Lösung von 13,43 g (50 mMol) Phosphorsäure-diphenylesterchlorid in 8 ml trockenem N,N-Dimethylformamid zu. Man rührt 20 Minuten im Aceton/Trockeneisbad und tropft dann bei -70° eine Lösung von 6,80 g (50 mMol) 3-Fluorbenzylisocyanid in 4 ml trockenem N,N-Dimethylformamid und anschliessend bei -70° bis -45° eine Lösung von 5,60 g (50 mMol) Kalium-t-butylat in 10 ml trockenem N,N-Dimethylformamid zu. Man lässt auf Raumtemperatur erwärmen und versetzt mit 2,5 ml Eisessig. Man giesst auf 300 ml Wasser und extrahiert fünfmal mit Methylenchlorid. Man wäscht die vereinigten organischen Auszüge dreimal mit Wasser, trocknet sie über Magnesiumsulfat und dampft ein. Durch Chromatographieren des Rückstands an Kieselgel unter Eluieren mit Essigester und anschliessende Umkristallisation aus Essigester erhält man (S)-8-Chlor-1-(m-fluorphenyl)-11,12,13, 13a-tetrahydro -9H-imidazo[1,5-a]pyrrolo[2,1-c][1,4]benzodiazepin-9-on vom Schmelzpunkt 239-240°.

Beispiel 4

Man versetzt eine Suspension von 6,49 g (30 mMol) (S)-1,2,3,11a-Tetrahydro-5H-pyrrolo[2,1-c][1,4]-benzodiazepin-5,11(10H)-dion in 30 ml trockenem N,N-Dimethylformamid bei -30° bis -20° mit 1,31 g (30 mMol) Natriumhydrid (55-proz. Oeldispersion), rührt noch während 35 Minuten bei obiger Temperatur und tropft dann bei -65° eine Lösung von 8,06 g (30 mMol) Phosphorsäure-diphenylesterchlorid in 6 ml trockenem N,N-Dimethylformamid zu. Man lässt auf -40° erwärmen, kühlt wieder ab gibt dann -70° eine

Lösung von 4,55 g (30 mMol) 3-Chlorbenzylisocyanid in 5 ml trockenem N,N-Dimethylformamid und anschliessend bei -70° bis -40° eine Lösung von 3,37 g (30 mMol) Kalium-t-butylat in 6 ml trockenem N,N-Dimethylformamid zu. Man lässt auf Raumtemperatur erwärmen und neutralisiert durch Zugabe von 1 ml Eisessig. Man giesst auf 250 ml Wasser und extrahiert fünfmal mit Methylenchlorid. Die vereinigten organischen Auszüge werden dreimal mit Wasser geswaschen, über Magnesiumsulfat getrocknet und eingedampft. Man chromatographiert den Rückstand unter Eluieren mit Essigester an Kieselgel und kristallisiert aus Essigester um. Man erhält (S)-1-(m-Chlorphenyl)-11,12,13,13a-tetrahydro -9H-imidazo[1,5-a]pyrrolo[2,1-c][1,4]benzodiazepin-9-on vom Schmelzpunkt 242-243°.

Beispiel 5

Man versetzt eine Suspension von 9,40 g (35 mMol) (S)-6-Chlor-7-fluor-1,2,3,11a-tetrahydro-5H -pyrrolo-[2,1-c][1,4]benzodiazepin-5,11(10H)-dion in 40 ml trockenem N,N-Dimethylformamid bei -30° bis -20° mit 1,53 g (35 mMol) Natriumhydrid (55-proz. Oeldispersion), rührt noch während 40 Minuten bei obiger Temperatur und tropft dann bei -65° eine Lösung von 9,40 g (35 mMol) Phosphorsäure-diphenylesterchlorid in 8 ml trockenem N,N-Dimethylformamid zu. Man lässt auf -40° erwärmen, kühlt wieder ab und tropft bei -65° eine Lösung von 5,03 g (35 mMol) 3-Chlorbenzylisocyanid in 5 ml trockenem N,N-Dimethylformamid und anschliessend bei -60° bis -40° eine Lösung von 3,92 g (35 mMol) Kalium-t-butylat in 10 ml trockenem N,N-Dimethylformamid zu. Man lässt auf Raumtemperatur erwärmen und neutralisiert mit 2 ml Eisessig. Man giesst auf 300 ml Wasser und extrahiert fünfmal mit Methylenchlorid. Man wäscht die vereinigten organischen Auszüge dreimal mit Wasser, trocknet sie über Magnesiumsulfat und dampft sie ein. Man chromatographiert den Rückstand unter Eluieren mit Essigester an Kieselgel und kristallisiert aus Essigester um. Man erhält (S)-8-Chlor-1-(m-chlorphenyl)-7-fluor-11,12,13,13a-tetrahydro -9H-imi-dazol[1,5-a]pyrrolo[2,1-c][1,4]benzodiazepin-9-on vom Schmelzpunkt 238-239°.

Beispiel 6

Man versetzt eine Suspension von 8,85 g (30 mMol) (S)-6-Brom-1,2,3,11a-tetrahydro -5H-pyrrolo[2,1-c]-[1,4]benzodiazepin-5,11(10H)-dion in 30 ml trockenem N,N-Dimethylformamid bei -30° bis -20° mit 1,31 g (30 mMol) Natriumhydrid (55-proz. Oeldispersion), rührt noch während 35 Minuten bei obiger Temperatur und tropft dann bei -65° eine Lösung von 8,06 g (30 mMol) Phosphorsäure-diphenylesterchlorid in 8 ml trockenem N,N-Dimethylformamid zu. Man lässt auf -40° erwärmen, kühlt wieder ab und tropft bei -65° eine Lösung von 4,55 g (30 mMol) 3-Chlorbenzylisocyanid in 5 ml trockenem N,N-Dimethylformamid und anschliessend bei -60° bis -40° eine Lösung von 3,37 g (30 mMol) Kalium-t-butylat in 7 ml trockenem N,N-Dimethylformamid zu. Man lässt auf Raumtemperatur erwärmen und neutralisiert durch Zugabe von 2 ml Eisessig. Man giesst auf 250 ml Wasser und extrahiert fünfmal mit Methylenchlorid. Man wäscht die vereinigten organischen Auszüge dreimal mit Wasser, trocknet sie über Magnesiumsulfat und dampft ein. Man chromatographiert den Rückstand unter Eluieren mit Essigester an Kieselgel und kristallisiert aus Essigester um. Man erhält (S)-8-Brom-1-(m-chlorphenyl)-11,12,13,13a-tetrahydro-9H -imidazo[1,5-a]pyrrolo-[2,1-c][1,4]benzodiazepin-9-on vom Schmelzpunkt 238-239°.

Beispiel 7

Man versetzt eine Suspension von 12,53 g (50 mMol) (S)-6-Chlor-1,2,3,11a-tetrahydro -5H-pyrrolo[2,1-c][1,4]-benzodiazepin-5,11(10H)-dion in 40 ml trockenem N,N-Dimethylformamid bei -30° bis -20° mit 2,18 g (50 mMol) Natriumhydrid (55-proz. Oeldispersion), rührt noch während 40 Minuten bei obiger Temperatur und tropft dann bei -70° eine Lösung von 13,43 g (50 mMol) Phosphorsäure-diphenylesterchlorid in 8 ml trockenem N,N-Dimethylformamid zu. Man rührt 20 Minuten im Aceton/Trockeneisbad und tropft dann bei -70° eine Lösung von 9,26 g (50 mMol) 3-Trifluormethyl-benzylisocyanid in 7 ml trockenem N,N-Dimethylformamid und anschliessend bei -70° bis -50° eine Lösung von 5,60 g (50 mMol) Kalium-t-butylat in 10 ml trockenem N,N-Dimethylformamid zu. Man lässt auf Raumtemperatur erwärmen und neutralisiert mit 2,5 ml Eisessig. Man giesst auf 300 ml Wasser und extrahiert fünfmal mit Methylenchlorid. Man wäscht die vereinigten organischen Auszüge dreimal mit Wasser, trocknet sie über Magnesiumsulfat und dampft ein. Der Rückstand wird unter Eluieren mit Essigester an Kieselgel chromatographiert und aus Essigester umkristallisiert. Man erhält (S)-8-Chlor-11,12,13,13a-tetrahydro-1-(α,α,α-trifluor -m-tolyl)-9H-imidazo[1,5-a]-pyrrolo[2,1-c][1,4]benzodiazepin -9-on vom Schmelzpunkt 202-204°.

Beispiel 8

Man versetzt eine Suspension von 8,20 g (35 mMol) (S)-7-Fluor-1,2,3,11a-tetrahydro-5H -pyrrolo[2,1-c]-[1,4]-benzodiazepin-5,11(10H)-dion in 40 ml trockenem N,N-Dimethylformamid bei -30° bis -20° mit 1,53 g (35 mMol) Natriumhydrid (55-proz. Oeldispersion), rührt noch während 35 Minuten bei obiger Temperatur und tropft dann bei -60° 7,25 ml (35 mMol) Phosphorsäure-diphenylesterchlorid in 7 ml trockenem N,N-Dimethylformamid zu. Man lässt die Temperatur auf -35° steigen, kühlt dann wieder auf -70° ab und gibt eine Lösung von 5,03 g (35 mMol) 3-Chlorbenzylisocyanid in 5 ml trockenem N,N-Dimethylformamid und anschliessend eine Lösung von 3,92 g (35 mMol) Kalium-t-butylat in 7 ml trockenem N,N-Dimethylformamid zu. Nach beendeter Zugabe lässt man auf 20° erwärmen, neutralisiert durch Zugabe von 1,5 ml Eisessig und giesst auf 250 ml Wasser. Man extrahiert fünfmal mit Methylenchlorid, wäscht die vereinigten organischen Auszüge dreimal mit Wasser, trocknet sie über Magnesiumsulfat und dampft ein. Man chromatographiert den Rückstand unter Eluieren mit Essigester an Kieselgel und kristallisiert aus Essigester und n-Hexan um. Man erhält (S)-1-(m-Chlorphenyl)-7-fluor-11,12,13,13a-tetrahydro-9H -imidazo[1,5-a]-pyrrolo[2,1-c][1,4]benzodiazepin-9-on vom Schmelzpunkt 182-183°.

Beispiel 9

10,03 g (40 mMol) (S)-6-Chlor-1,2,3,11a,-tetrahydro-5H-pyrrolo[2,1-c][1,4]benzodiazepin-5,11(10H)-dion werden in 35 ml trockenem N,N-Dimethylformamid suspendiert und bei -30° bis -20° mit 1,75 g (40 mMol) Natriumhydrid (55-proz. Oeldispersion) versetzt. Man rührt noch 35 Minuten bei -30° bis -20°, tropft anschliessend bei -60° eine Lösung von 8,3 ml (40 mMol) Phosphorsäure-diphenylesterchlorid in 8 ml trockenem N,N-Dimethylformamid zu und lässt auf -35° erwärmen. Man kühlt wieder auf -70° ab und gibt eine Lösung von 7,80 g (40 mMol) 3-Brombenzylisocyanid in 4 ml trockenem N,N-Dimethylformamid und anschliessend eine Lösung von 4,50 g (40 mMol) Kalium-t-butylat in 10 ml trockenem N,N-Dimethylformamid zu. Nach beendeter Zugabe lässt man auf 20° erwärmen, neutralisiert mit 1 ml Eisessig und giesst auf 250 ml Wasser. Man extrahiert fünfmal mit Methylenchlorid, wäscht die vereinigten organischen Auszüge dreimal mit Wasser, trocknet sie über Magnesiumsulfat und dampft ein. Man chromatographiert den Rückstand unter Eluieren mit Essigester an Kieselgel und kristallisiert anschliessend aus Essigester um. Man erhält (S)-1-(m-Bromphenyl)-8-chlor-11,12,13,13a-tetrahydro-9H -imidazo[1,5-a]pyrrolo[2,1-c][1,4]-benzodiazepin-9-on vom Schmelzpunkt 247-248°.

Beispiel 10

Man versetzt eine Suspension von 8,17 g (36,8 mMol) (S)-5a,6,7,8a-Tetrahydro-5H -pyrrolo[1,2-a]thieno-[3,2-e][1,4]diazepin-5,10(4H)-dion in 35 ml trockenem N,N-Dimethylformamid bei -30° bis -20° mit 1,60 g (36,8 mMol) Natriumhydrid (55-proz. Oeldispersion), rührt während 45 Minuten bei obiger Temperatur und tropft dann bei -70° eine Lösung von 9,90 g (36,8 mMol) Phosphorsäure-diphenylesterchlorid in 7 ml trockenem N,N-Dimethylformamid zu. Man lässt auf -40° erwärmen, kühlt wieder ab und gibt bei -70° eine Lösung von 7,21 g (36,8 mMol) 3-Brombenzylisocyanid in 4 ml trockenem N,N-Dimethylformamid und anschliessend bei -70° bis -40° eine Lösung von 4,13 g (36,8 mMol) Kalium-t-butylat in 10 ml trockenem N,N-Dimethylformamid zu. Man lässt auf Raumtemperatur erwärmen und neutralisiert durch Zugabe von 2 ml Eisessig. Man giesst auf 400 ml Wasser und extrahiert viermal mit Methylenchlorid. Man wäscht die organischen Auszüge viermal mit Wasser, trocknet sie über Magnesiumsulfat und dampft sie ein. Man chromatographiert den Rückstand an Kieselgel unter Eluieren mit Essigester und kristallisiert anschliessend aus Essigester und Hexan um. Man erhält (S)-1-(m-Bromphenyl)-10,11,12,12a-tetrahydro-8H -imidazo[5,1-c]pyrrolo[1,2-a]thieno[3,2-e][1,4]diazepin-8-on vom Schmelzpunkt 169-170°.

Beispiel 11

Man versetzt eine Suspension von 11,83 g (50 mMol) (S)-5-Chlor-1,10a-dihydro-azeto[2,1-c][1,4]-benzodiazepin -4,10(2H,9H)-dion in 35 ml trockenem N,N-Dimethylformamid bei -30° bis -20° mit 2,18 g (50 mMol) Natriumhydrid (55-proz. Oeldispersion), rührt noch während 35 Minuten bei obiger Temperatur und tropft dann bei -70° eine Lösung von 13,43 g (50 mMol) Phosphorsäure-diphenylesterchlorid in 8 ml trockenem N,N-Dimethylformamid zu. Nach 20-minütigem Rühren im Aceton/Trockeneisbad tropft man bei -70° eine Lösung von 7,58 g (50 mMol) 3-Chlorbenzylisocyanid in 4 ml trockenem N,N-Dimethylformamid und anschliessend bei -65° bis -40° eine Lösung von 5,61 g (50 mMol) Kalium-t-butylat in 9 ml trockenem N,N-Dimethylformamid zu. Man lässt auf Raumtemperatur erwärmen und neutralisiert durch Zugabe von 2,5 ml Eisessig. Man giesst auf 300 ml Wasser und extrahiert fünfmal mit Methylenchlorid. Die vereinigten organischen Auszüge werden dreimal mit Wasser gewaschen, über Magnesiumsulfat getrocknet und

eingeengt. Man chromatographiert den Rückstand an Kieselgel unter Eluieren mit Essigester und kristallisiert zweimal aus Essigester um. Man erhält (S)-8-Chlor-1-(m-chlorphenyl)-12,12a-dihydro-9H -azeto[2,1-c]-imidazo[1,5-a][1,4]benzodiazepin-9-on vom Schmelzpunkt 221-223°.

Beispiel 12

Man versetzt eine Suspension von 12,53 g (50 mMol) (S)-6-Chlor-1,2,3,11a-tetrahydro-5H -pyrrolo[2,1-c][1,4]benzodiazepin-5,11(10H)-dion in 50 ml trockenem N,N-Dimethylformamid bei -30° bis -20° mit 2,07 g (47,5 mMol) Natriumhydrid (55-proz. Oeldispersion), rührt noch während 30 Minuten bei obiger Temperatur und tropft dann bei -65° eine Lösung von 12,89 g (48 mMol) Phosphorsäure-diphenylesterchlorid in 6 ml trockenem N,N-Dimethylformamid zu. Man rührt während 20 Minuten im Aceton/Trockeneisbad und gibt dann bei -70° eine Lösung von 7,58 g (50 mMol) 2-Chlorbenzylisocyanid in 5 ml trockenem N,N-Dimethylformamid und anschliessend bei -70° bis -40° eine Lösung von 5,61 g (50 mMol) Kalium-t-butylat in 10 ml trockenem N,N-Dimethylformamid zu. Man lässt auf Raumtemperatur erwärmen und neutralisiert mit 2 ml Eisessig. Man giesst auf 300 ml Wasser und extrahiert fünfmal mit Methylenchlorid. Man wäscht die vereinigten organischen Auszüge dreimal mit Wasser, trocknet sie über Magnesiumsulfat und dampft ein. Durch Chromatographieren des Rückstands an Kieselgel unter Eluieren mit Essigester und anschliessende Umkristallisation aus Essigester und n-Hexan erhält man (S)-8-Chlor-1-(o-chlorphenyl)-11,12,13,13a-tetrahydro-9H -imidazo[1,5-a]pyrrolo[2,1-c][1,4]benzodiazepin-9-on vom Schmelzpunkt 246-247°.

Verbindungen der eingangs definierten Formel I, wie (S)-8-Chlor-1-(m-chlorphenyl)-11,12,13,13a-tetrahydro-9H -imidazo[1,5-a]pyrrolo[2,1-c][1,4]benzodiazepin-9-on, können wie im nachstehenden Beispiel A angegeben, als Wirkstoffe zur Herstellung von pharmazeutischen Präparaten verwendet werden.

Beispiel A

Es werden in üblicher Weise Tabletten folgender Zusammensetzung hergestellt:

| | mg/Tablette |
|---|---|
| Wirkstoff | 5 |
| Milchzucker | 140 |
| Maisstärke | 46 |
| Polyvinylpyrrolidin | 8 |
| Magnesiumstearat | 1 |
| Tablettengewicht | 200 |

## Ansprüche

1. Imidazodiazepin-Derivate der allgemeinen Formel

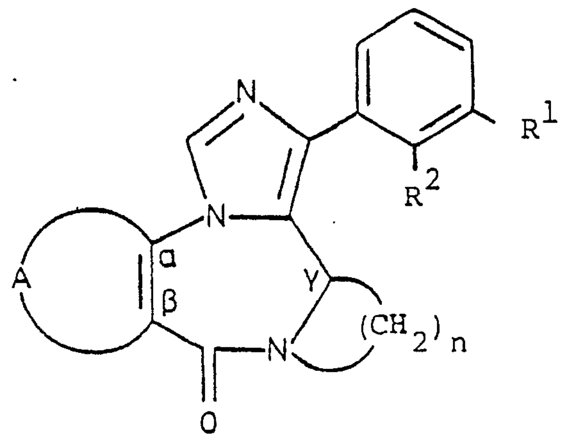

I

worin A zusammen mit den mit α und ß bezeichneten Kohlenstoffatomen die Gruppe

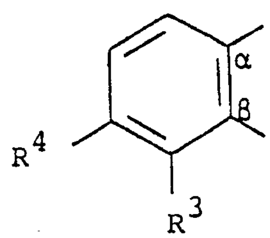

(a)

oder

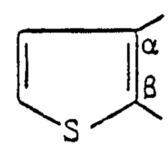

(b)

eines von $R^1$ und $R^2$ Wasserstoff und das andere Wasserstoff, Halogen oder Trifluormethyl, $R^3$ und $R^4$ je Wasserstoff oder Halogen und n die Zahl 2 oder 3 bedeuten, wobei die Verbindungen bezüglich des mit γ bezeichneten Kohlenstoffatoms die (S)- oder (R,S)-Konfiguration aufweisen, und pharmazeutisch annehmbare Säureadditionssalze davon.

**2.** Verbindungen gemäss Anspruch 1, worin A die Gruppe (a), $R^3$ Halogen und $R^4$ Wasserstoff bedeuten.

**3.** Verbindungen gemäss Anspruch 2, worin $R^3$ Chlor oder Brom bedeutet.

**4.** Verbindungen gemäss einem der Ansprüche 1 bis 3, worin $R^1$ Halogen oder Trifluormethyl und $R^2$ Wasserstoff bedeuten.

**5.** Verbindungen gemäss Anspruch 4, worin $R^1$ Fluor, Chlor, Brom oder Trifluormethyl bedeutet.

**6.** Verbindungen gemäss einem der Ansprüche 1 bis 5, worin das mit γ bezeichnete Kohlenstoffatom die (S)-Konfiguration aufweist.

**7.** (S)-8-Chlor-1-(m-chlorphenyl)-11,12,13,13a-tetrahydro-9H-imidazo[1,5-a]pyrrolo[2,1-c][1,4]-benzodiazepin-9-on.

**8.** (S)-8-Chlor-1-(m-fluorphenyl)-11,12,13,13a-tetrahydro-9H-imidazo[1,5-a]pyrrolo[2,1-c][1,4]-benzodiazepin-9-on.

**9.** (S)-8-Brom-1-(m-chlorphenyl)-11,12,13,13a-tetrahydro-9H-imidazo[1,5-a]pyrrolo[2,1-c][1,4]-benzodiazepin-9-on.

**10.** (S)-8-Chlor-11,12,13,13a-tetrahydro-1-phenyl-9H-imidazo[1,5-a]pyrrolo[2,1-c][1,4]benzodiazepin-9-on, (S)-1-(m-Chlorphenyl)-11,12,13,13a-tetrahydro-9H-imidazo-[1,5-a]pyrrolo[2,1-c][1,4]benzodiazepin-9-on,
(S)-8-Chlor-1-(m-chlorphenyl)-7-fluor-11,12,13,13a-tetrahydro-9H-imidazo-[1,5-a]pyrrolo[2,1-c][1,4]-benzodiazepin-9-on,
(S)-8-Chlor-11,12,13,13a-tetrahydro-1-(α,α,α-trifluor-m-tolyl)-9H-imidazo-[1,5-a]pyrrolo[2,1-c][1,4]-benzodiazepin-9-on,
(S)-1-(m-Chlorphenyl)-7-fluor-11,12,13,13a-tetrahydro-9H-imidazo-[1,5-a]pyrrolo[2,1-c][1,4]-benzodiazepin-9-on,
(S)-1-(m-Bromphenyl)-8-chlor-11,12,13,13a-tetrahydro-9H-imidazo-[1,5-a]pyrrolo[2,1-c][1,4]-benzodiazepin-9-on,
(S)-1-(m-Bromphenyl)-10,11,12,12a-tetrahydro-8H-imidazo-[5,1-c]pyrrolo[1,2-a]thieno[3,2-e][1,4]-diazepin-8-on,
(S)-8-Chlor-1-(m-chlorphenyl)-12,12a-dihydro-9H-azeto-[2,1-c]imidazo[1,5-a][1,4]benzodiazepin-9-on und
(S)-8-Chlor-1-(o-chlorphenyl)-11,12,13,13a-tetrahydro-9H-imidazo-[1,5-a]pyrrolo[2,1-c][1,4]-benzodiazepin-9-on.

**11.** Verbindungen gemäss einem der Ansprüche 1 bis 10 zur Anwendung als therapeutische Wirkstoffe.

**12.** Verbindungen gemäss einem der Ansprüche 1 bis 10 zur Anwendung als anxiolytische, antikonvulsive, muskelrelaxierende und sedativ-hypnotische Wirkstoffe.

**13.** Verfahren zur Herstellung von Verbindungen gemäss einem der Ansprüche 1 bis 10, dadurch gekennzeichnet, dass man eine Verbindung der allgemeinen Formel

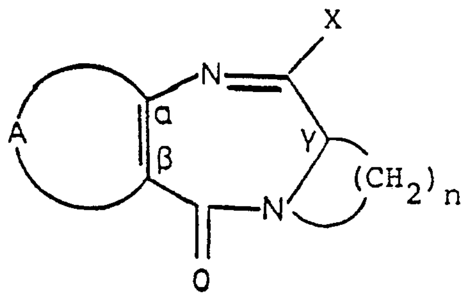

II

worin A und n die in Anspruch 1 angegebene Bedeutung besitzen und X eine Abgangsgruppe bedeutet,
in Gegenwart einer Base mit einem Isonitril der allgemeinen Formel

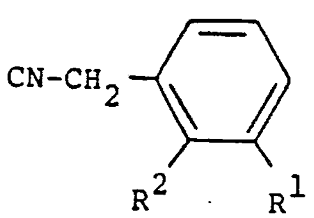

III

worin $R^1$ und $R^2$ die in Anspruch 1 angegebene Bedeutung besitzen,
umsetzt und erwünschtenfalls eine erhaltene Verbindung der allgemeinen Formel I in ein pharmazeutisch annehmbares Säureadditionssalz überführt.

**14.** Arzneimittel, enthaltend eine Verbindung gemäss einem der Ansprüche 1 bis 10 und ein therapeutisch inertes Excipiens.

**15.** Antikonvulsiv, anxiolytisch, muskelrelaxierend und sedativ-hypnotisch wirksame Arzneimittel gemäss Anspruch 14.

16. Verwendung von Verbindungen gemäss einem der Ansprüche 1 bis 10 zur Herstellung von antikonvulsiv, anxiolytisch, muskelrelaxierend und sedativ-hypnotisch wirksamen Arzneimitteln.

Patentansprüche für folgenden Vertragsstaat: AT

1. Verfahren zur Herstellung von Imidazodiazepin-Derivaten der allgemeinen Formel

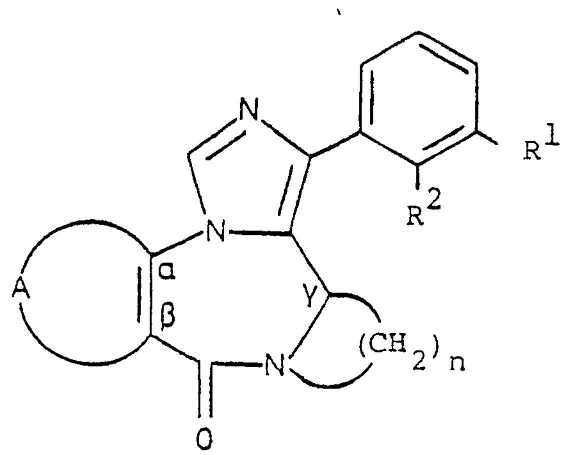

I

worin A zusammen mit den mit $\alpha$ und $\beta$ bezeichneten Kohlenstoffatomen die Gruppe

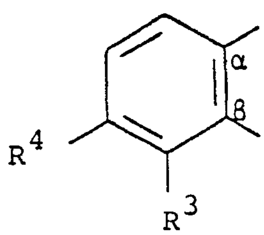

(a)

oder

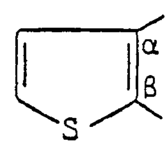

(b)

eines von $R^1$ und $R^2$ Wasserstoff und das andere Wasserstoff, Halogen oder Trifluormethyl, $R^3$ und $R^4$ je Wasserstoff oder Halogen und n die Zahl 2 oder 3 bedeuten, wobei die Verbindungen bezüglich des mit $\gamma$ bezeichneten Kohlenstoffatoms die (S)- oder (R,S)-Konfiguration aufweisen,
und von pharmazeutisch annehmbaren Säureadditionssalzen davon, dadurch gekennzeichnet, dass man eine Verbindung der allgemeinen Formel

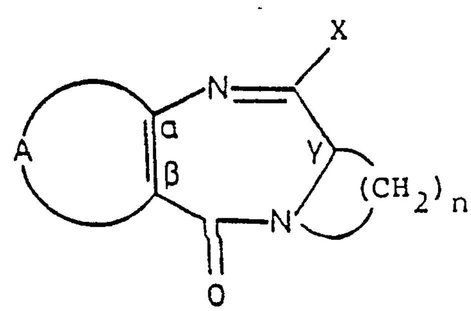

II

worin A und n obige Bedeutung besitzen und X eine Abgangsgruppe bedeutet,
in Gegenwart einer Base mit einem Isonitril der allgemeinen Formel

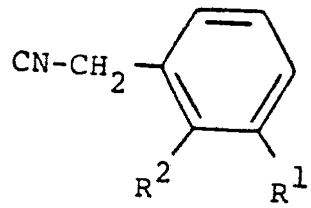

III

worin $R^1$ und $R^2$ obige Bedeutung besitzen,
umsetzt und erwünschtenfalls eine erhaltene Verbindung der allgemeinen Formel I in ein pharmazeutisch annehmbares Säureadditionssalz überführt.

2. Verfahren gemäss Anspruch 1, worin A die Gruppe (a), $R^3$ Halogen und $R^4$ Wasserstoff bedeuten.

3. Verfahren gemäss Anspruch 2, worin $R^3$ Chlor oder Brom bedeutet.

4. Verfahren gemäss einem der Ansprüche 1 bis 3, worin $R^1$ Halogen oder Trifluormethyl und $R^2$ Wasserstoff bedeuten.

5. Verfahren gemäss Anspruch 4, worin $R^1$ Fluor, Chlor, Brom oder Trifluormethyl bedeutet.

6. Verfahren gemäss einem der Ansprüche 1 bis 5, worin das mit $\gamma$ bezeichnete Kohlenstoffatom die (S)-Konfiguration aufweist.

7. Verfahren gemäss Anspruch 1, dadurch gekennzeichnet, dass man (S)-8-Chlor-1-(m-chlorphenyl)-11,12,13,13a-tetrahydro-9H-imidazo[1,5-a]pyrrolo[2,1-c][1,4]benzodiazepin-9-on herstellt.

8. Verfahren gemäss Anspruch 1, dadurch gekennzeichnet, dass man (S)-8-Chlor-1-(m-fluorphenyl)-11,12,13,13a-tetrahydro-9H-imidazo[1,5-a]pyrrolo[2,1-c][1,4]benzodiazepin-9-on herstellt.

9. Verfahren gemäss Anspruch 1, dadurch gekennzeichnet, dass man (S)-8-Brom-1-(m-chlorphenyl)-11,12,13,13a-tetrahydro-9H-imidazo[1,5-a]pyrrolo[2,1-c][1,4]benzodiazepin-9-on herstellt.

## Claims

1. Imidazodiazepine derivatives of the general formula

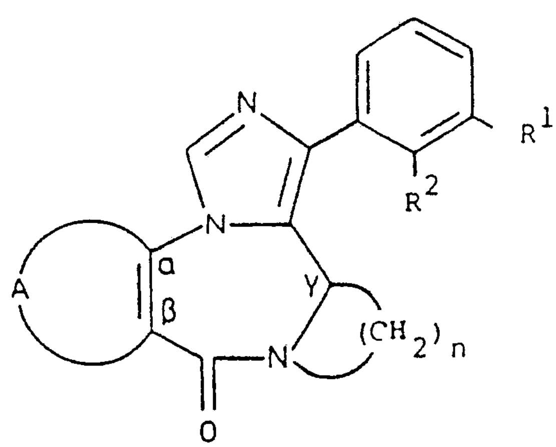

I

wherein A together with the carbon atoms denoted by $\alpha$ and $\beta$ signifies the group

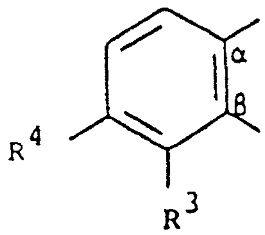

or

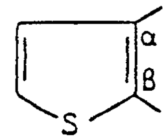

(a) (b)

one of $R^1$ and $R^2$ signifies hydrogen and the other signifies hydrogen, halogen or trifluoromethyl, $R^3$ and $R^4$ each signify hydrogen or halogen and n signifies the number 2 or 3, the compounds having the (S)- or (R,S)-configuration with reference to the carbon atom denoted by $\gamma$,
and pharmaceutically acceptable acid addition salts thereof.

**2.** Compounds in accordance with claim 1, wherein A signifies the group (a), $R^3$ signifies halogen and $R^4$ signifies hydrogen.

**3.** Compounds in accordance with claim 2, wherein $R^3$ signifies chlorine or bromine.

**4.** Compounds in accordance with any one of claims 1 to 3, wherein $R^1$ signifies halogen or trifluoromethyl and $R^2$ signifies hydrogen.

**5.** Compounds in accordance with claim 4, wherein $R^1$ signifies fluorine, chlorine, bromine or trifluoromethyl.

**6.** Compounds in accordance with any one of claims 1 to 5, wherein the carbon atom denoted by $\gamma$ has the (S)-configuration.

**7.** (S)-8-Chloro-1-(m-chlorophenyl)-11,12,13,13a-tetrahydro-9H-imidazo[1,5-a]pyrrolo[2,1-c][1,4]-benzodiazepin-9-one.

**8.** (S)-8-Chloro-1-(m-fluorophenyl)-11,12,13,13a-tetrahydro-9H-imidazo[1,5-a]]pyrrolo[2,1-c][1,4]-benzodiazepin-9-one.

**9.** (S)-8-Bromo-1-(m-chlorophenyl)-11,12,13,13a-tetrahydro-9H-imidazo[1,5-a]pyrrolo[2,1-c][1,4]-benzodiazepin-9-one.

**10.** (S)-8-Chloro-11,12,13,13a-tetrahydro-1-phenyl-9H--imidazo[1,5-a]pyrrolo[2,1-c][1,4]benzodiazepin-9-one,
(S)-1-(m-chlorophenyl)-11,12,13,13a-tetrahydro-9H--imidazo[1,5-a]pyrrolo[2,1-c][1,4]benzodiazepin-9-one,
(S)-8-chloro-1-(m-chlorophenyl)-7-fluoro-11,12,13,13a-tetrahydro-9H-imidazo[1,5-a]pyrrolo[2,1-c]-[1,4]benzodiazepin-9-one,
(S)-8-chloro-11,12,13,13a-tetrahydro-1-($\alpha$,$\alpha$,$\alpha$--trifluoro-m-tolyl)-9H-imidazo[1,5-a]pyrrolo[2,1-c][1,4]-benzodiazepin-9-one,
(S)-1-(m-chlorophenyl)-7-fluoro-11,12,13,13a-tetrahydro-9H-imidazo[1,5-a]pyrrolo[2,1-c][1,4]-benzodiazepin-9-one,
(S)-1-(m-bromophenyl)-8-chloro-11,12,13,13a-tetrahydro-9H-imidazo[1,5-a]pyrrolo[2,1-c][1,4]-benzodiazepin-9-one,
(S)-1-(m-bromophenyl)-10,11,12,12a-tetrahydro-8H-imidazo[5,1-c]pyrrolo[1,2-a]thieno[3,2-e][1,4]-diazepin-8-one,
(S)-8-chloro-1-(m-chlorophenyl)-12,12a-dihydro-9H-azeto[2,1-c]imidazo[1,5-a][1,4]benzodiazepin-9-one and
(S)-8-chloro-1-(o-chlorophenyl)-11,12,13,13a-tetrahydro-9H-imidazo[1,5-a]pyrrolo[2,1-c][1,4]-benzodiazepin-9-one.

**11.** Compounds in accordance with any one of claims 1 to 10 for use as therapeutically active substances.

**12.** Compounds in accordance with any one of claims 1 to 10 for use as anxiolytic, anticonvulsant, muscle relaxant and sedative-hypnotic active substances.

**13.** A process for the manufacture of compounds in accordance with any one of claims 1 to 10, characterized by reacting a compound of the general formula

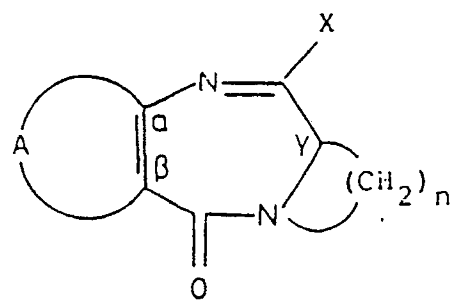

II

wherein A and n have the significance given in claim 1 and X signifies a leaving group, in the presence of a base with an isonitrile of the general formula

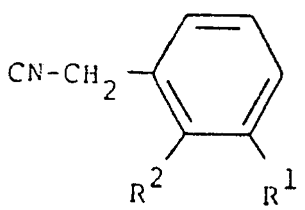

III

wherein $R^1$ and $R^2$ have the significance given in claim 1, and, if desired, converting a compound of general formula I obtained into a pharmaceutically acceptable acid addition salt.

**14.** A medicament containing a compound in accordance with any one of claims 1 to 10 and a therapeutically inert excipient.

**15.** A medicament having anticonvulsant, anxiolytic, muscle relaxant and sedative-hypnotic activity in accordance with claim 14.

**16.** The use of compounds in accordance with any one of claims 1 to 10 for the manufacture of medicaments having anticonvulsant, anxiolytic, muscle relaxant and sedative-hypnotic activity.

Claims for the following Contracting State: AT

**1.** A process for the manufacture of imidazodiazepine derivatives of the general formula

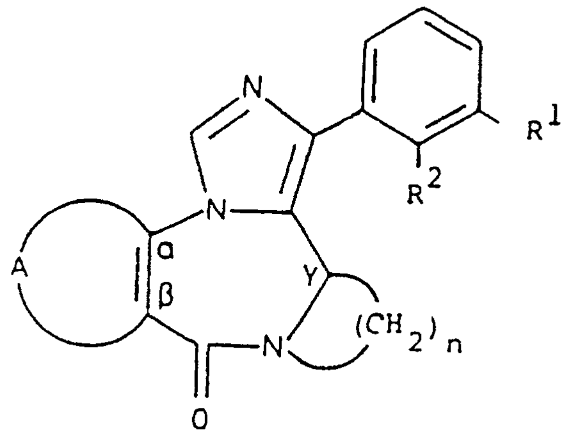

I

wherein A together with the carbon atoms denoted by α and β signifies the group

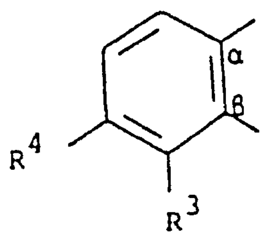

or

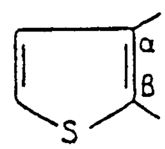

(a) (b)

one of $R^1$ and $R^2$ signifies hydrogen and the other signifies hydrogen, halogen or trifluoromethyl, $R^3$ and $R^4$ each signify hydrogen or halogen and n signifies the number 2 or 3, the compounds having the (S)- or (R,S)-configuration with reference to the carbon atom denoted by γ,
and of pharmaceutically acceptable acid addition salts thereof, characterized by reacting a compound of the general formula

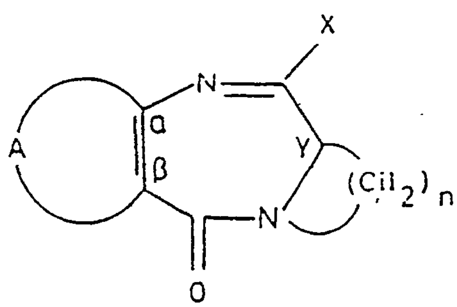

II

wherein A and n have the above significance and X signifies a leaving group,
in the presence of a base with an isonitrile of the general formula

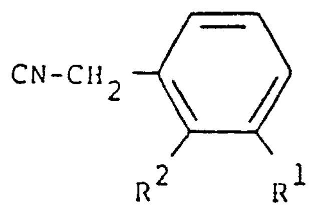

III

wherein $R^1$ and $R^2$ have the above significance, and, if desired, converting a compound of general formula I obtained into a pharmaceutically acceptable acid addition salt.

2. A process in accordance with claim 1, wherein A signifies the group (a), $R^3$ signifies halogen and $R^4$ signifies hydrogen.

3. A process in accordance with claim 2, wherein $R^3$ signifies chlorine or bromine.

4. A process in accordance with any one of claims 1 to 3, wherein $R^1$ signifies halogen or trifluoromethyl and $R^2$ signifies hydrogen.

5. A process in accordance with claim 4, wherein $R^1$ signifies fluorine, chlorine, bromine or trifluoromethyl.

6. A process in accordance with any one of claims 1 to 5, wherein the carbon atom denoted by $\gamma$ has the (S)-configuration.

7. A process in accordance with claim 1, characterized in that (S)-8-chloro-1-(m-chlorophenyl)-11,12,13,13a-tetrahydro-9H-imidazo[1,5-a]pyrrolo[2,1c]-[1,4]benzodiazepin-9-one is manufactured

8. A process in accordance with claim 1, characterized in that (S)-8-chloro-1-(m-fluorophenyl)-11,12,13,13a-tetra-hydro-9H-imidazo[1,5-a]]pyrrolo[2,1-c]-[1,4]benzodiazepin-9-one is manufactured.

9. A process in accordance with claim 1, characterized in that (S)-8-bromo-1-(m-chlorophenyl)-11,12,13,13a-tetrahydro-9H-imidazo[1,5-a]pyrrolo[2,1-c]-[1,4]benzodiazepin-9-one is manufactured.

## Revendications

1. Dérivés de l'imidazodiazépine de formule générale

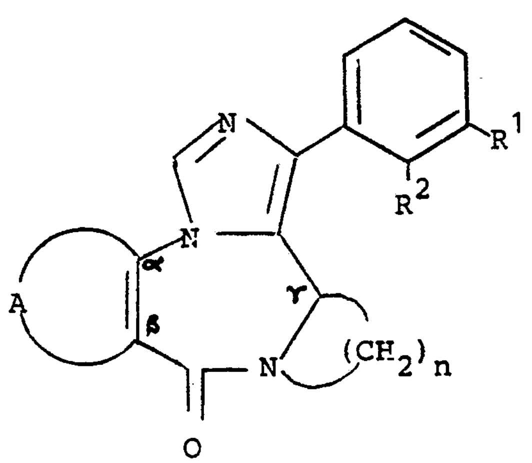

I

dans laquelle A forme avec les atomes de carbone $\alpha$ et $\beta$ le groupe

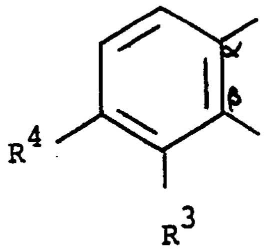

ou

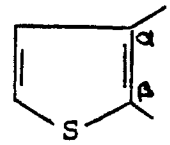

(a) (b)

l'un des symboles $R^1$ et $R^2$ représente l'hydrogène et l'autre l'hydrogène, un halogène ou un groupe trifluorométhyl $R^3$ et $R^4$ représentent chacun l'hydrogène ou un halogène e n est égal à 2 ou 3, les composés étant en configuration (S) ou (R,S) par rapport à l'atome de carbone marqué $\gamma$, et leurs sels formés par addition avec des acides acceptables pour l'usage pharmaceutique.

2. Composés selon la revendication 1, dans lesquels A représente le groupe (a), $R^3$ un halogène et $R^4$ l'hydrogène.

3. Composés selon la revendication 2, dans lesquels $R^3$ représente le chlore ou le brome.

4. Composés selon l'une des revendications 1 à 3, dans lesquels $R^1$ représente un halogène ou le groupe trifluorométhyle et $R^2$ l'hydrogène.

5. Composés selon la revendication 4, dans lesquels $R^1$ représente le fluor, le chlore, le brome ou un groupe trifluorométhyle.

6. Composés selon l'une des revendications 1 à 5, dans lesquels l'atome de carbone $\gamma$ est en configuration S.

7. La (S)-8-chloro-1-m-chlorophényl)-11,12,13,13a-tétrahydro-9H-imidazo∠1,5-a7pyrrolo∠2,1-c7∠1,47benzodiazépine-9-one.

8. La (S)-8-chloro-1-(m-fluorophényl)-11,12,13,13a-tétrahydro-9H-imidazo∠1, 5-a7pyrrolo∠2,1-c7∠1,47benzodiazépine-9-one.

9. La (S)-8-bromo-1-(m-chlorophényl)-11,12,13,13a-tétrahydro-9H-imidazo∠1,5-a7pyrrolo∠2,1-c7∠1,47benzodiazépine-9-one.

10. La (S)-8-chloro-11,12,13,13a-tétrahydro-1-phényl-9H-imidazo∠1,5-a7pyrrolo∠2,1-c7∠1,47benzodiazépine-9-one,
la (S)-1-(m-chlorophényl)-11,12,13,13a-tétrahydro-9H-imidazo∠1,5-a7pyrrolo∠2,1-c7∠1,47benzodiazépine-9-one,
la (S)-8-chloro-1-(m-chlorophényl)-7-fluoro-11,12, 13,13a-tétrahydro-9H-imidazo-∠1,5-a7pyrrolo∠2,1-c7∠1,47-benzodiazépine-9-one,
la (S)-8-chloro-11,12,13,13a-tétrahydro-1-($\alpha,\alpha,\alpha$-trifluoro-m-tolyl)-9H-imidazo-∠1,5-a7pyrrolo∠2,1-c7∠1,47-benzodiazépine-9-one,
la (S)-1-(m-chlorophényl)-7-fluoro-11,12,13,13a-tétrahydro-9H-imidazo-∠1,5-a7pyrrolo/2,1-c7/1,47benzodiazépine-9-one,
la (S)-1-(m-bromophényl)-8-chloro-11,12,13,13a-tétrahydro-9H-imidazo-∠1,5-a7pyrrolo∠2,1-c7∠1,47benzodiazépine-9-one,
la (S)-1-(m-bromophényl)-10,11,12,12a-tétrahydro-8H-imidazo∠5,1-c7pyrrolo∠1,2-a7thiéno∠3,2-e7∠1,47diazépine-8-one,
la (S)-8-chloro-1-(m-chlorophényl)-12,12a-dihydro-9H-azéto∠2,1-c7imidazo∠1,5-a7∠1,47benzodiazéine-9-one et

la (S)-8-chloro-1-(o-chlorophényl)-11,12,13,13a-tétrahydro-9H-imidazo-. [1,5-a]pyrrolo[2,-c][1,4]benzodiazépine-9-one.

11. Composés selon l'une des revendications 1 à 10, pour l'utilisation en tant que substances actives thérapeutiques.

12. Composés selon l'une des revendications 1 à 10, pour l'utilisation en tant que substances actives anxiolytiques, anticonvulsives, myorelaxantes et sédatives-hypnotiques.

13. Procédé de préparation des composés selon l'une des revendications 1 à 10, caractérisé en ce que l'on fait réagir un composé de formule générale

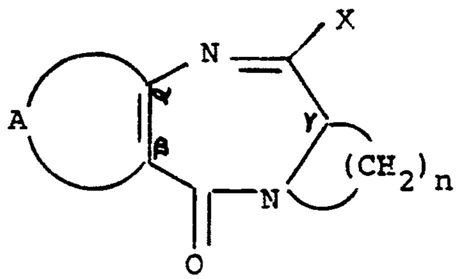

II

dans laquelle A et n ont les significations indiquées dans la revendication 1 et X représente un groupe éliminable,
en présence d'une base, avec un isonitrile de formule générale

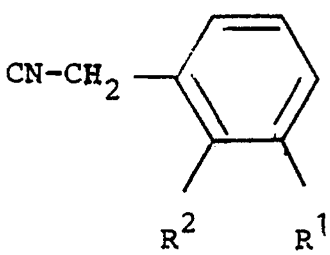

III

dans laquelle $R^1$ et $R^2$ ont les significations indiquées dans la revendication 1, et si on le désire, on convertit un composé ainsi obtenu, répondant à la formule générale I, en un sel par addition avec un acide acceptable pour l'usage pharmaceutique.

14. Médicament contenant un composé selon l'une des revendications 1 à 10 et un excipient inerte du point de vue thérapeutique.

15. Médicament à activité anticonvulsive, anxiolytique, myorelaxante et sédative-hypnotique selon la revendication 14.

16. Utilisation des composés selon l'une des revendications 1 à 10 pour la préparation de médicaments à activité anticonvulsive, anxiolytique, myorelaxante et sédative-hypnotique.

Revendications pour l'Etat contractant suivant: AT

1. Procédé de préparation de dérivés de l'imidazodiazépine de formule générale

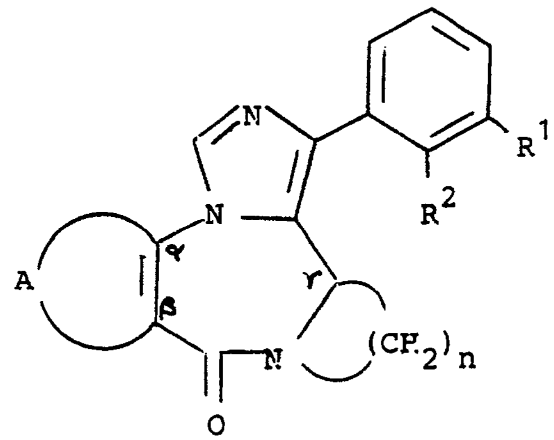

I

dans laquelle A forme avec les atomes de carbone α et ß le groupe

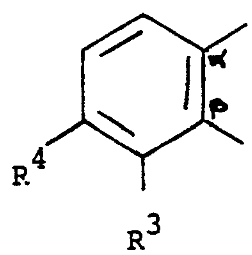

(a)

ou

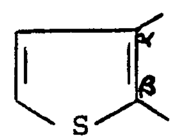

(b)

l'un des symboles $R^1$ et $R^2$ représente l'hydrogène et l'autre l'hydrogène, un halogène ou un groupe trifluorométhyle, $R^3$ et $R^4$ représentent chacun l'hydrogène ou un halogène et n est égal à 2 ou 3, les composés étant en configuration S ou R,S par rapport à l'atome de carbone γ,
et de leurs sels formés par addition avec des acides acceptables pour l'usage pharmaceutique, caractérisé en ce que l'on fait réagir un composé de formule générale

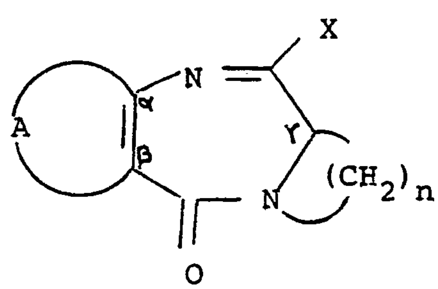

II

dans laquelle A et n ont les significations indiquées ci-dessus et X représente un groupe éliminable, en présence d'une base, avec un isonitrile de formule générale

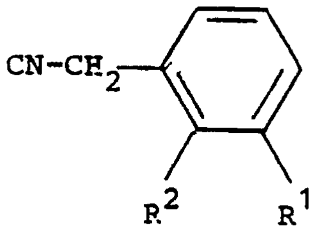

III

dans laquelle $R^1$ et $R^2$ ont les signifi cations indiquées ci-dessus,
et si on le désire, on convertit un composé ainsi obtenu, répondant à la formule générale I, en un sel formé par addition avec un acide acceptable pour l'usage pharmaceutique.

**2.** Procédé selon la revendication 1, dans lequel A représente le groupe (a), $R^3$ un halogène et $R^4$ l'hydrogène.

**3.** Procédé selon la revendication 2, dans lequel $R^3$ représente le chlore ou le brome.

**4.** Procédé selon l'une des revendications 1 à 3, dans lequel $R^1$ représente un halogène ou un groupe trifluorométhyle et $R^2$ l'hydrogène.

**5.** Procédé selon la revendication 4, dans lequel $R^1$ représente le fluor, le chlore, le brome ou un groupe trifluorométhyle.

**6.** Procédé selon l'une des revendications 1 à 5, dans lequel l'atome de carbone $\gamma$ est en configuration S.

**7.** Procédé selon la revendication 1, caractérisé en ce que l'on prépare la (S)-8-chloro-l-(m-chlorophényl)-11,12,13,13a-tétrahydro-9H-imidazo[1,5-a]pyrrolo[2,1-c][1,4]benzodiazépine-9-one.

**8.** Procédé selon la revendication 1, caractérisé en ce que l'on prépare la (S)-8-chloro-1-(m-fluorophényl)-11,12,13,13a-tétrahydro-9H-imidazo[1,5-a]pyrrolo[2,1-c][1,4]benzodiazépine-9-one.

**9.** Procédé selon la revendication 1, caractérisé en ce que l'on prépare la (S)-8-bromo-1-(m-chlorophényl)-11,12,13,13a-tétrahydro-9H-imidazo[1,5-a]pyrrolo[2,1-c][1,4]benzodiazépine-9-one.